# EUROPEAN PATENT SPECIFICATION

(11) **EP 1 588 686 B1**
(45) Date of publication and mention of the grant of the patent: **18.08.2010**
(21) Application number: 04425281.5
(22) Date of filing: 23.04.2004
(51) Int. Cl.: A61K 8/87, A61K 8/89, A61Q 1/02, A61Q 1/06, A61Q 1/08, A61Q 1/10

(54) **Cosmetic compositions and their use**
Kosmetische Zusammensetzungen und deren Verwendung
Compositions cosmétiques et leurs utilisations.

(43) Date of publication of application: 26.10.2005
(73) Proprietor: Intercos S.p.A., 20122 Milano (IT)
(72) Inventor: Maio, Giuseppe, 20080 Zelo Surrigone MI (IT); Rando, Pietro, 20090 Opera MI (IT)
(74) Representative: Mittler, Enrico

(56) References cited:
- EP-A- 1 410 782
- WO-A-03/040214
- DE-A- 10 223 693
- DE-A- 10 223 694
- US-A- 5 993 972
- US-A- 6 107 352
- DATABASE REGISTRY [Online] CHEMICAL ABSTRACTS SERVICE, COLUMBUS, OHIO, US; retrieved from stn Database accession no. 156327-07-0

## Description

### Field of the invention

The present invention relates to cosmetic compositions comprising a silicone-containing polyurethane and to a process for preparing said silicone-containing polyurethane.

More particularly it relates to a cosmetic composition comprising a particular class of silicone-containing polyurethanes with particular cosmetic properties, especially for the skin, hair, nails, lips, eyelashes etc, and to a process for preparing said silicone-containing polyurethanes.

### Background of the invention

Cosmetic products for the make-up of the face, the lips, the eyelashes etc often suffer from the drawback that, when they come into contact with e. g. the fingers or clothing, they tend to smudge or soil these surfaces. In addition, in some cases the make-up can also appear to be not homogeneous. Consequently, in order to avoid these problems cosmetic products which have high adhesive properties and which provide for the deposition of a homogeneous, long-lasting film onto e. g. the facial skin, the lips, the eyelashes etc are of particular interest.

Usually, the formulator attempts to achieve these properties by including a functional film-forming polymer in the cosmetic product. Such polymer must also be physiologically compatible with the skin and offer protection against dehydration, UV light and so on.

However, the Applicant has found that commonly used functional film-forming polymers proved to be unsuitable for application to the lips, as the film tended to crack down under the normal and continuous lips' movement. Moreover, toughness of the film often caused a feeling of discomfort when applied onto the lips or onto the skin.

It is, therefore, an object of the present invention to overcome these drawbacks and to provide a cosmetic composition comprising a particular silicone-containing polyurethane with improved film-forming, adhesive and sensorial properties.

### Description of the related art

It has already been proposed in the art to use silicone-containing polyurethanes as film-forming polymers in cosmetic compositions for the skin, hair, eyelashes etc. Thus,US-A-6,166,093 (Mougin, et al.) describes the use of a polyurethane block polycondensation product comprising a polysiloxane graft for treating keratinous materials. The polysiloxane oligomer has a diol or diamine function at only one end of its chain.

A similar technology is described in US-A- 6,319,959(Mougin, et al.).

In US-A- 5,643,581 (Mougin, et al.) a cosmetic composition for the skin, hair, lips etc is described which contains a multiblock polycondensate of a polyurethane and a polysiloxane, wherein the polyurethane further comprises anionic or cationic groups.

In US-A- 2002076425 (= FR 2,814,365;Mondet, et al.) a cosmetic composition is described which contains a polyurethane with at least two urethane groups and at least one hydrocarbon-based unit chosen from hydrocarbon blocks and grafts or esters thereof. These polymers may additionally contain a polyorganosiloxane.

EP-A- 1 410 782 discloses a film agent made of an amphoteric urethane resin and cosmetics containing the same. In particular, comparative Example 8 describes the production of a polysiloxane-carring amphiphilic urethane resin (T) using the same procedure as described in the Production Example 1 except of using 8 g KF 6002 and 55 g of the polypropylene glycol instead of 8 g of the dimethylpolysiloxane and 63 g of the polypropylene glycol, respectively.

KF 6002 has been described In the Registry Number 156327-07-0 from the registry file, Chemical Abstracts Service as an alpha-[3-(2-hydroxyethoxy)propyl]dimethylsilyl]-omega-[[[3-(2-hdroxyethoxy)propylidimethylsilyl]oxy]-dimethylpolysiloxane.

In EP-A- 1 410 782 an aqueous liquid of an amphiphilc urethane resin (T) has been obtained.

US-A-5,993,972 discloses amphiphilic polyether polyurethanes made from a hydrophilic and a hydrophobic diol, and optionally a polydimethylsiloxane polyoxyalkylene copolymer.

DE-A-10223693 describes sunscreen compositions with sillcone-IPDI polymers, in which the silicone is a dimethiconol or a dimethiconol/dimethicone copolymer.

WO 03/040214 describes aromatic substituted polysiloxane prepolymers for biocompatible polymers for ophthalmic devices.

Finally, dimethiconol- or dimethicone-containing polyurethanes are known cosmetic ingredients, see e.g.US-A-20020028875 (Anderle, et al.) and US-A- 6,120,753 (Vinski, et al.). Dimethiconol-containing polyurethanes are commercially available from ALZO,Inc under the tradename Polyderm PPI-SI Despite these prior proposals there is still a need for a cosmetic composition having high film-forming and adhesive properties together with improved sensorial properties of smoothness and comfort, and it is an object of the present invention to provide such a composition.

### Summary of the invention

According to the present invention, this object is achieved by providing a cosmetic composition comprising a particular silicone-containing polyurethane with a molecular weight of between 40,000 and 400,000, and particularly preferably between 100,000 and 280,000. The particular silicone, used as pre-polymer in the polyurethane of the invention is

α,ω-di(2-propoxyethanol) polydimethylsiloxane (= polydimethylsiloxane, 2-propoxyethanol terminated), having about 20 silyloxy units in its chain.

A preferred polyurethane according to the present invention, having a molecular weight of between 100,000 and 280,000, is the reaction product of this preferred silicone pre-polymer with isophorone diisocyanate

### Detailed Description Of The Invention

The amount of the silicone-containing polyurethane used in the cosmetic compositions ranges from 0.25 to 40% by weight of the composition, preferably from 0.5 to 30% and particularly preferably from 1 to 20%.

The polyurethane of the invention can be suitably prepared by the process as described in claim 1.

The final polymer is then produced by the reaction of the quasi-polymer with a chain extender, also known as a curative. Chain extenders are polyfunctional chemicals such as monoalcohols, polyalcohols, dicarboxylic acids and so on. The chain extender zips up the quasi-polymer molecules, thus increasing the molecular weight and creating the final polymer. The chain extenders play an important role in the synthesis as many of the actual cosmetic and rheological properties of the polymer depend on the appropriate choice of the chain extender.

The polyurethanes of the present invention preferably contain a chain extender.

### Diisocyanates

The diisocyanates, suitable for use in the manufacture of the polyurethanes of the present invention are isophorone diisocyanate, lysine alkyl ester diisocyanate and arginine alkyl ester diisocyanate, wherein the alkyl group may be methyl, ethyl, propyl and butyl and mixture thereof.

### Chain extenders

If the polyurethanes of the present invention also contain a chain extender - which is a preferred embodiment of the invention - the choice of the chain extender determines the characteristics of the polymer's chain and the behaviour in cosmetic formulations. Thus, simple diols yield linear polyurethanes, whilst triols or polyols create highly branched polyurethanes. The structure of the polyurethane has great influence on its physical behaviour when it is used in cosmetic formulations. Thus e.g. the viscosity of its solution can be tuned by varying the degree of branching.

The chain extender also carries in the polymer its whole structure besides the mere alcoholic or carboxylic functionality needed for the chain extension. This means that the molecule required as chain extender can be chosen according to the cosmetic properties which the formulator wishes to impart to a cosmetic product. Thus e.g. the lipophylicity of the polyurethane can be tuned by introducing different long carbon chain 1,2-diols or different fatty acid monoglycerides.

Particularly suitable chain extenders were found to be C8-C28 fatty acid esters of polyhydroxy compounds such as glycerol, polyglycerol, pentaerythritol, sucrose, glucose, fructose, sorbitan etc. A preferred extender of this class is sorbitan monostearate.

Other suitable extenders are esters of C8-C28 fatty alcohols with hydroxyacids such as lactic acid, maleic acid, tartaric acid, citric acid. Non-limitative examples are stearyl tartrate, C12-C13 alkyl malate, C12-C13 alkyl lactate, C14-C15 alkyl citrate.

Other suitable extenders were found to be acyl-aminoacids derived from glutamic acid, leucine, arginine, cysteine, lysine, serine, threonine, tyrosine, hydroxyproline, ornithine, citrulline, homocysteine, homoserine, cystine, statine with (C8-C28) acyl groups such as stearoyl glutamate and stearoyl leucine.

Yet another group of suitable extenders are vitamins as such or C2-C28 alkyl esters or ethers of vitamins, such as esters or ethers of vitamin B1, B2, B5, B6, and C.

### Catalysts

In the above step-polymerization process a catalyst is desirable to shorten the reaction time. Well-known catalysts are tertiary amines such as DABCO (bicycloamine) or triethylamine, which work better on aromatic isocyanates. Metal Lewis acids, such as tin, bismuth, iron or zinc derivatives efficiently catalyze reaction with both aliphatic and aromatic isocyanates. We have found that for cosmetic purposes the best catalysts are zinc salts of long-chain(C8-C28) fatty acids ("zinc soaps"), such as zinc laurate, zinc myristate, zinc palmitate, zinc oleate, zinc linoleate, zinc linolenate and particularly zinc stearate. Zinc stearate has the advantage that for cosmetic purposes it does not have to be removed from the reaction mixture.

### Reaction solvent

The above polymerization process is usually carried out in a solvent. Many solvents are known for use in this process.

Light paraffins and isoparaffins with from 8-24 carbon atoms in their alkyl chain were found to be most attractive, such as decane, isodecane , isododecane and isohexadecane , and mixtures of various isoparaffins. The preferred solvent is isododecane.

### Quenching

After complete polymerization, any unreacted isocyanate group is converted into urethane by adding an alcohol such as ethanol.

### Reaction condition

By balancing the degree of polymerization, the degree of branching, the molecular weight and the percentage of the polyurethane in the solvent solution, the proper viscosity for cosmetic use can be obtained.

The molecular weight can be measured by means of Size Exclusion Chromatography (SEC) or by means of Gel Permeation Chromatography (GPC), a method which uses High Performance Layer Chromatography (HPLC) with an isocratic pump, a refraction index detector and a column thermostat at 25° C. The measurement is performed by dissolving 10-50mg of the polymer sample in 1 ml THF at room temperature. Subsequently, with a suitable column (e. g. from Polymer Laboratories PL gel) and the calibration curve as function of the expected molecular weight, the chromatographic conditions set up during the calibration phase are maintained (THF mobile phase at room temperature 1mL/min). Once the test samples are injected, the results are evaluated with suitable GPC software to extrapolate the molecular weight.

The viscosity of a 25-27% by weight solution of the polyurethane of the invention in isododecane can be measured with Brookfield viscosimeter DV-1, with R2 spindle at 20 rpm and at 25°C. For optimum results the polyurethane solution of the invention should have a viscosity of between 1,000 and 10,000, preferably between 1,000 and 6,000, and particularly preferably between 1,300 and 2,500 mPa.s at 25°C (+/- 0.1).

### Further ingredients of the cosmetic composition

The composition of the invention may furthermore comprise clays, waxes, solvents, silicones, cosmetic excipients, colourants, preservatives, (co)polymers (other than the polyurethanes of the invention) e.g. polyisoprene, fragrances, flavours, vitamins, antioxidants, vegetable or mineral oils and fats, pearlescent agents, surface-active agents etc. Clays, waxes and solvents are particularly useful further ingredients and are discussed in more detail hereunder.

### Clays

The composition of the invention may contain a clay, either unmodified or modified. Typical examples of unmodified clays are smectite clays such as hectorites, montmorillonites and bentonites. Modified clays are clays, which have been made oleophilic by treating them with a cationic compound. Such clays are known in the art. Typical examples are smectite clays such as hectorites, montmorillonites and bentonites, which have been made oleophilic by treating them with an organic cationic compound. Typical examples of the oleophilic modified clays are stearalkonium bentonite, and preferably disteardimonium hectorite. The amount of the clay, when used in the present invention, ranges from 0.05- 20% by weight of the composition, and preferably from 0.1-10% by weight.

### Solvents

The composition of the invention may contain a solvent It can be any organic solvent, suitable for use in cosmetic products. Typical examples are aliphatic hydrocarbons with from 8 to 24 carbon atoms, such as isoparaffins like isooctane, isononane, isodecane, isododecane, Isopars (RTM) ex Exxon, etc. Isododecane is the preferred solvent.

The solvent can be used in the present invention in an amount of between 1.1 - 90% by weight of the composition, preferably 10-80% by weight of the composition.

Furthermore, the composition may also preferably contain a silicone , such as (cyclo) polysiloxanes e. g. cyclomethicone and/or dimethicone , in an amount of between 0. 5 - 20% by weight of the composition.

### Waxes

The composition of the invention may also comprise a wax, such as candelilia wax, carnauba wax, beeswax, ceresine, microcrystalline wax, paraffin wax, silicone wax, polyethylene wax and the like in an amount of between 0.5 - 20% by weight of the composition.

### Excipients and colorants

The balance of the composition contains the usual cosmetic excipients, colourants and other additives in an amount of between 1.1 % -80%, preferably 5-20% by weight of the composition. Suitable cosmetic excipients are e. g. talc, mica, silicas, kaolin, zinc oxide, calcium carbonate, magnesium carbonate phosphate, starch and its derivatives, nylon, polyethylene, acrylic (co) polymers and so on. Suitable colourants are e. g. iron oxides, chromium oxide and/or hydroxide, blue and pink ultramarine, manganese violet, titanium dioxide, pearlescent pigments based on mica or bismuth oxychloride substrates, carmin lakes and pigments based on organic colorants as listed by the CTFA.

Lipophilic (co)polymers derived from e. g. polyvinylpyrrolidone, from fluor-containing monomers, from acrylic monomers etc, may also be used in the composition of the invention in an amount of between 1 - 20% by weight of the composition. These lipophilic (co)polymers may even enhance the film-forming action of the polyurethane of the invention.

The composition of the invention may be in liquid, semiliquid, paste-like or cake- or other solid form.

The composition of the invention may be made in any convenient way. A suitable way is first to prepare a dispersion of the polyurethane in the organic solvent, and subsequently adding to the resulting semiliquid mixture the other components of the composition.

The invention will be further illustrated by the following non-limitative examples. The aliquots used in the reactions were at least stoichiometric.

### EXAMPLES

### Example 1

A suitable aliquot of neat siliconic polyol (α,ω-di(2-propoxyethanol)polydimethylsiloxane (=polydimethylsiloxane, 2-propoxyethanol terminated) was loaded into a stainless steel reactor with stirring. The polyol was diluted with an aliquot of isododecane and the reactor was flushed with nitrogen. The whole reaction was run under nitrogen. A suspension of an aliquot of zinc stearate catalyst in isododecane was added through a dropping funnel. The dropping funnel was rinsed with a suitable aliquot of isododecane which was then added into the reactor. An aliquot of neat |PD| (isophorone diisocyanate) was added into the reactor and the dropping funnel was rinsed with an aliquot of isododecane which was then added into the reactor.

The solution was heated at 95° C under reflux for 15 hours, then cooled at 70° C and diluted with an aliquot of isododecane. An aliquot of ethyl alcohol was then added and the solution was heated at 80° C for 3 hours. The absence of free NCO groups was checked by FTIR and the solution was cooled at 50° C and filtered through a stainless steel sieve. A cloudy solution of the silicone-containing polyurethane in isododecane was obtained. When a neat sample was required, the solvent was removed under reduced pressure and a sticky sample of the crude polyurethane was obtained. The polyurethane had a molecular weight of between 180,000 and 200,000 , and a viscosity of between 1,300 and 1,800 mPa.s at a concentration of 25-27% in isododecane and at a temperature of 25°C.

### Example 2

Neat siliconic polyol (same as in example 1) was loaded into a stainless steel reactor under stirring. The polyol was diluted with an aliquot of isododecane and the reactor was flushed under nitrogen. The whole reaction was run under nitrogen. A suspension of an aliquot of zinc stearate catalyst in suitable aliquot of isododecane was added through a dropping funnel. The dropping funnel was rinsed with isododecane which was then added to the reactor. A suitable aliquot of neat IPDI (isophorone diisocyanate) was added to the reactor and the dropping funnel was rinsed with isododecane which was then added to the reactor.

The solution was heated at 95°C under reflux for 3 hours, and then mixed with a solution of a suitable aliquot of sorbitan monostearate in isododecane which has been previously heated at 95°C for 30 minutes.

After adding the sorbitan stearate solution, the whole reaction was carried out for 10 hours at 95°C still under nitrogen reflux.

After 10 hours, a second solution of an aliquot of neat |PD| in isododecane was added to the reactor through the dropping funnel which was then rinsed with isododecane and the reaction was carried out for further 5 hours, still at 95°C and under nitrogen.

The whole solution was then cooled at 70°C and suitable aliquot of ethyl alcohol was added to terminate the reaction (to ensure the blocking of isocyanate groups statistically present at one or both ends of the polymer which were then transformed into ethyl urethane) and heated at 80°C for 3 hours.

The absence of free NCO groups was checked by FTIR and the solution was cooled at 50°C and filtered through a stainless steel sieve.

A cloudy solution of sugar ester silicone/urethane copolymer in isododecane was obtained, the viscosity of which was in the range of 3,000-4,000 mPa*s at a concentration of 25-27% by weight. The molecular weight was 220,000 - 260,000. The solvent was removed under reduced pressure and a sticky sample of the crude polyurethane was obtained.

### Example 3

A suitable aliquot of neat siliconic polyol (same as in example 1) was loaded into a stainless steel reactor under stirring. The polyol was diluted with a suitable aliquot of Isododecane and the reactor was flushed under nitrogen. The whole reaction was run under nitrogen. A suspension of a suitable aliquot of zinc stearate catalyst in isododecane was added through a dropping funnel. The dropping funnel was rinsed with an aliquot of isododecane which was then added to the reactor. A suitable aliquot of neat IPDI (isophorone diisocyanate) was added to the reactor and the dropping funnel was rinsed with an aliquot of isododecane which was then added to the reactor.

The solution was heated at 95°C under reflux for 3 hours, and then mixed with a solution of a suitable aliquot of sorbitan monostearate in isododecane which has been previously heated at 95°C for 30 minutes.

After adding the sorbitan monostearate solution, the whole reaction was carried out for 5 hours at 95°C still under nitrogen reflux.

A second solution of a suitable aliquot of ethyl panthenol in isododecane was added to the reactor through the dropping funnel which was then rinsed with isododecane and the reaction was carried out for further 4 hours, still at 95°C and under nitrogen.

The whole solution was then cooled at 70°C and a suitable aliquot of ethyl alcohol was added to terminate the reaction (to ensure the blocking of isocyanate groups statistically present at one or both ends of the polymer which were then transformed into ethyl urethane) and heated at 80°C for 3 hours.

The absence of free NCO groups was checked by FTIR and the solution was cooled at 50°C and filtered through a stainless steel sieve.

A cloudy solution of sugar ester panthenol-silicone/urethane copolymer in isododecane was obtained, the viscosity of which was in the range of 4,000-5,000 mPa*s at concentration of about 25-27% by weight. The molecular weight was 280.000 and 320.000. The solvent was removed under reduced pressure and a sticky sample of the crude polyurethane was obtained.

### Example 4

A suitable aliquot of neat siliconic polyol (same as in example 1) was loaded into a stainless steel reactor under stirring. The polyol was diluted with an aliquot of Isododecane and the reactor was flushed under nitrogen. The whole reaction was run under nitrogen. A suspension of a suitable aliquot of zinc stearate catalyst in isododecane was added through a dropping funnel. The dropping funnel was rinsed with an aliquot of isododecane which was then added to the reactor. A suitable aliquot of neat ethyl lysine diisocyanate was added to the reactor and the dropping funnel was rinsed with isododecane, which was then added to the reactor.

The solution was heated at 95°C under reflux for 3 hours, and then mixed with a solution of a suitable aliquot of sorbitan monostearate in isododecane, which has been previously heated at 95°C for 30 minutes.

After adding the sorbitan monostearate solution, the whole reaction was carried out for 10 hours at 95°C still under nitrogen reflux.

The whole solution was then cooled at 70°C and a suitable aliquot of ethyl alcohol was added to terminate the reaction (to ensure the blocking of isocyanate groups statistically present at one or both ends of the polymer which were then transformed into ethyl urethane) and heated at 80°C for 3 hours.

The absence of free NCO groups was checked by FTIR and the solution was cooled at 50°C and filtered through a stainless steel sieve.

A cloudy solution of sugar ester silicone/urethane copolymer in isododecane was obtained, the viscosity of which was in the range of 3,200 - 4,200 mPa*s at a concentration of about 25-27% by weight. The molecular weight was 240.000 and 270.000. The solvent was removed under reduced pressure and a sticky sample of the crude polyurethane was obtained.

The following Examples are examples of compositions according to the invention.

### Example 5 (Lip colouring fluid)

| **ingredient** | **% weight** |
|---|---|
| Isododecane | 61.00 |
| Silicone Polyurethane Ex.1 | 10.00 |
| Disteardimonium Hectorite | 8.50 |
| Propylene Carbonate | 2.00 |
| Alcohol | 0.80 |
| D&C Red 7 Ca Lake | 0.70 |
| Titanium Dioxide | 1.40 |
| Iron Oxide Yellow | 1.15 |
| Fd&C Blue 1 Al Lake | 0.50 |
| Iron Oxide Red | 0.85 |
| Silica | 0.30 |
| Dimethicone | 10.00 |
| Mica And Titanium Dioxide | 2.60 |
| Flavour | 0.20 |
| TOTAL | **100.00** |

### Example 6 (Lip colouring fluid)

| **Ingredient** | **% weight** |
|---|---|
| Isododecane | 75.45 |
| Silicone Polyurethane Ex.2 | 8.00 |
| Disteardimonium Hectorite | 6.00 |
| Propylene Carbonate | 1.50 |
| Alcohol | 0.50 |
| D&C Red 7 Ca Lake | 0.70 |
| Titanium Dioxide | 1.40 |
| Iron Oxide Yellow | 1.15 |
| Fd&C Blue 1 Al Lake | 0.40 |
| Iron Oxide Red | 0.85 |
| Silica | 0.30 |
| Mica And Titanium Dioxide | 2.60 |
| Flavour | 0.10 |
| Tocopheryl Linoleate | 0.05 |
| TOTAL | **100.00** |

### Example 7 (Mascara)

| **Ingredient** | **% weight** |
|---|---|
| Isododecane | 57.0 |
| Cyclomethicone | 10.0 |
| Propylsilsesquioxane | 5.0 |
| Hydrogenated Polyisobutene | 7.0 |
| Silicone Polyurethane Ex. 1 | 3.5 |
| Iron Oxide Black | 7.0 |
| Sucrose Stearate | 1.5 |
| Disteardimonium Hectorite | 0.8 |
| Propylene carbonate | 0.2 |
| TOTAL | 100.0 |

### Example 8 (Lipstick)

| **Ingredient** | **% weight** |
|---|---|
| Isododecane | 22.95 |
| Polyethylene | 23.00 |
| Cyclopentasiloxane And Polypropylsiloxane | 13.00 |
| Diisostearyl Malate | 3.00 |
| Polymethylsilsesquioxane | 3.50 |
| Silicone Polyurethane Ex.1 | 6.00 |
| Propylparaben | 0.20 |
| BHT | 0.02 |
| Mica And Titanium Dioxide | 10.20 |
| Iron Oxide Red | 3.40 |
| Iron Oxide Yellow | 0.50 |
| Titanium Dioxide | 1.50 |
| Disteardimonium Hectorite | 0.15 |
| Propylene Carbonate | 0.05 |
| Glycerin | 1.03 |
| Coconut Oil And Tiare' Flower | 0.50 |
| TOTAL | **100.00** |

### Example 9 (Face Fluid Foundation)

| **Ingredient** | **% weight** |
|---|---|
| Microcrystalline Wax | 1.20 |
| Laureth-9 | 0.55 |
| Polyglyceryl-4 Isostearate | 0.82 |
| Isododecane | 30.43 |
| Silicone Polyurethane Ex.4 | 5.50 |
| Sodium Chloride | 1.50 |
| Water | 40.00 |
| D&C Red 7 Ca Lake | 0.70 |
| Titanium Dioxide | 8.60 |
| Iron Oxide Yellow | 3.20 |
| Iron Oxide Black | 0.10 |
| Iron Oxide Yellow | 1.40 |
| Iron Oxide Black | 0.30 |
| Iron Oxide Red | 3.50 |
| Glycerine | 2.00 |
| Fragrance | 0.20 |
| TOTAL | **100.00** |

### Example 10 (Powder Eye Shadow)

| **Ingredient** | **% weight** |
|---|---|
| Isododecane | 2.40 |
| Talc | 59.50 |
| Mica | 4.00 |
| OctyIDodecylStearoylStearate | 2.60 |
| Dimethicone | 1.30 |
| Silicone Polyurethane Ex. 1 | 0.80 |
| Iron Oxide Black | 0.10 |
| Iron Oxide Yellow | 8.00 |
| Iron Oxide Red | 3.00 |
| Pearl (Mica/Titanium) | 8.00 |
| Pearl (Mica/TiO2/Iron Oxide) | 10.00 |
| Preservatives | 0.30 |
| TOTAL | **100.0** |

### Example 11 (Compact Powder)

| **Ingredient** | **% weight** |
|---|---|
| Isododecane | 3.00 |
| Talc | 80.45 |
| Mica | 8.00 |
| OctyIDodecylStearoyiStearate | 1.70 |
| Dimethicone | 0.80 |
| Silicone Polyurethane Ex. 3 | 1.00 |
| Iron Oxide Black | 0.15 |
| Iron Oxide Yellow | 1.50 |
| Iron Oxide Red | 0.60 |
| Pearl (Mica/Titanium) | 1.00 |
| Nylon 12 | 1.50 |
| Preservatives | **0.30** |
| TOTAL | **100.0** |

## Claims

1. A cosmetic composition with improved skin substantive, long wear and non-transfer properties comprising:
- 0.25%-40% by weight of the composition of a particular silicone-containing polyurethane, in which the silicone-containing polyurethane is obtainable by the process comprising the step of reacting a siliconol and a diisocyanate in a solvent in the presence of a catalyst wherein the siliconol is α,ω-di(2-propoxyethanol)polydimethylsiloxane having about 20 silyloxy units in its chain, the diisocyanate is selected from the group consisting of isophorone diisocyanate, lysine ester diisocyanate and arginine ester diisocyanate and the solvent is isododecane, the silicone-containing polyurethane having a molecular weight of between 40,000 and 400,000,
- the balance comprising conventional cosmetic excipients, colourants and additives.

2. A composition according to claim 1, **characterized in that** the molecular weight of the silicone-containing polyurethane ranges from 100,000 and 280,000.

3. A composition according to claim 1 , **characterized in that** it comprises from 0.25% -40% of the silicone-containlng polyurethane.

4. A composition according to claim 3, **characterized in that** it comprises from 0.5%-30% of the silicone-containing polyurethane.

5. A composition according to claim 4, **characterized in that** it comprises from 1%-20% of the silicone-containing polyurethane.

6. A composition according to claim 1, **characterized in that** the silicone-containing polyurethane also contains a chain extender.

7. A composition according to claim 6, **characterized in that** the chain extender is selected from the group consisting of polyols, C8-C28 fatty acid esters of polyols, hydroxyacids, acylaminoacids, vitamins and vitamin esters.

8. A composition according to claim 7, **characterized in that** the chain extender is sorbitan monostearate.

9. Use of a silicone-containing polyurethane as defined in claims 1-8 as film-forming agent in cosmetic products for the skin, eyes, lips or keratinous materials.

## Patentansprüche

1. Kosmetische Zusammensetzung mit verbesserten Eigenschaften bezüglich Hautanhaftung, Langzeittragefähigkeit und Verschmierungsresistenz, umfassend:
- 0,25 - 40 Gewichts-% der Zusammensetzung eines speziellen silikonhaltigen Polyurethans, wobei das silikonhaltige Polyurethan erhältlich ist durch ein Verfahren, das den Schritt des Umsetzens eines Siloxans und eines Diisocyanats in einem Lösungsmittel in Gegenwart eines Katalysators umfasst, wobei das Siloxan α,ω-di(2-Propoxyethanol)polydimethylsiloxan mit etwa 20 Silyloxy-Gruppen in der Polymerkette ist, das Diisocyanat ausgewählt ist aus der Gruppe bestehend aus Isophorondiisocyanat, Lysinesterdiisocyanat und Argininesterdilsocyanat und das Lösungsmittel Isododekan ist, wobei das silikonhaltige Polyurethan ein Molekulargewicht zwischen 40000 und 400000 hat,
- wobei der Rest herkömmliche kosmetische Trägerstoffe, Farbstoffe and Zusatzmittel umfasst.

2. Zusammensetzung nach Anspruch 1, **dadurch gekennzeichnet, dass** das Molekulargewicht des silikonhaltigen Polyurethans im Bereich von 100000 bis 280000 liegt.

3. Zusammensetzung nach Anspruch 1, **dadurch gekennzeichnet, dass** sie 0,25 - 40% des silikonhaltigen Polyurethans umfasst.

4. Zusammensetzung nach Anspruch 3, **dadurch gekennzeichnet, dass** sie 0,5 - 30% des silikonhaltigen Polyurethans umfasst.

5. Zusammensetzung nach Anspruch 4, **dadurch gekennzeichnet, dass** sie 1 - 20% des silikonhaltigen Polyurethans umfasst,

6. Zusammensetzung nach Anspruch 1, **dadurch gekennzeichnet, dass** das silikonhaltige Polyurethan auch einen Kettenverlängerer enthält.

7. Zusammensetzung nach Anspruch 6, **dadurch gekennzeichnet, dass** der Kettenverlängerer ausgewählt ist aus der Gruppe bestehend aus Polyolen, C8 - C28 Fettsäureestern von Polyolen, Hydroxysäuren, Acylaminosäuren, Vitaminen und Vitaminestern.

8. Zusammensetzung nach Anspruch 7, **dadurch gekennzeichnet, dass** der Kettenverlängerer Sorbitanmonostearat ist.

9. Verwendung des silikonhaltigen Polyurethans wie in Ansprüchen 1 bis 8 definiert als Film bildendes Mittel in kosmetischen Produkten für die Haut, Augen, Lippen oder kreatinhaltige Materialien.

## Revendications

1. Composition cosmétique ayant des propriétés améliorées de substantivité cutanée, de résistance à l'usure et de non-transfert, comprenant :
- 0,25 % à 40 % en poids de la composition d'un polyuréthane particulier contenant de la silicone, où le polyuréthane contenant de la silicone est susceptible d'être obtenu par le procédé comprenant l'étape de mise à réagir d'un siliconol avec un diisocyanate dans un solvant en présence d'un catalyseur, où le siliconol est le α,ω-di(2-propoxy-éthanol)-polydiméthylsiloxane ayant environ 20 motifs silyloxy dans sa chaîne, le diisocyanate est sélectionné dans le groupe constitué du diisocyanate d'isophorone, du diisocyanate ester de lysine et du diisocyanate ester d'arginine et le solvant est l'isododécane, le polyuréthane contenant de la silicone ayant une masse moléculaire comprise entre 40 000 et 400 000,
- le reste comprenant des excipients, des colorants et des additifs cosmétiques classiques.

2. Composition selon la revendication 1, **caractérisée en ce que** la masse moléculaire du polyuréthane contenant de la silicone est comprise entre 100 000 et 280 000.

3. Composition selon la revendication 1, **caractérisée en ce qu'**elle comprend de 0,25 % à 40 % du polyuréthane contenant de la silicone.

4. Composition selon la revendication 3, **caractérisée en ce qu'**elle comprend de 0,5 % à 30 % du polyuréthane contenant de la silicone.

5. Composition selon la revendication 4, **caractérisée en ce qu'**elle comprend de 1 % à 20 % du polyuréthane contenant de la silicone.

6. Composition selon la revendication 1, **caractérisée en ce que** le polyuréthane contenant de la silicone contient également un allongeur de chaîne.

7. Composition selon la revendication 6, **caractérisée en ce que** l'allongeur de chaîne est sélectionné dans le groupe constitué des polyols, des esters d'acides gras en C8-C28 de polyols, des hydroxyacides, des acides acylaminés, des vitamines et des esters de vitamines.

8. Composition selon la revendication 7, **caractérisée en ce que** l'allongeur de chaîne est le monostéarate de sorbitan.

9. Utilisation d'un polyuréthane contenant de la silicone selon les revendications 1 à 8 comme agent filmogène dans des produits cosmétiques destinés à la peau, aux yeux ou aux lèvres, ou des matières kératiniques.
